# EUROPEAN PATENT SPECIFICATION

(11) **EP 1 951 699 B1**
(45) Date of publication and mention of the grant of the patent: **10.10.2012**
(21) Application number: 06826616.2
(22) Date of filing: 26.10.2006
(51) Int. Cl.: C07D 295/192

(54) **PROCESSES FOR THE PREPARATION OF CYCLOPROPYL-AMIDE DERIVATIVES**
VERFAHREN ZUR HERSTELLUNG VON CYCLOPROPYLAMIDDERIVATEN
PROCÉDÉS POUR LA SYNTHÈSE DE DÉRIVÉS DE CYCLOPROPYLAMIDES

(30) Priority: 31.10.2005 US 731725 P
(43) Date of publication of application: 06.08.2008
(73) Proprietor: Janssen Pharmaceutica NV, 2340 Beerse (BE)
(72) Inventor: MANI, Neelakandha, S., San Diego, California 92129 (US); PALMER, David, C., Doylestown, Pennsylvania 18901 (US); PANDIT, Chennagiri, R., San Diego, California 92129 (US); REYES, Mayra, B., Somerset, New Jersey 08873 (US); XIAO, Tong, Edison, New Jersey 08820 (US); CESCO-CANCIAN, Sergio, Bethlehem, PA 18020 (US)
(74) Representative: Warner, James Alexander
(86) International application number: PCT/US2006/041590
(87) International publication number: WO 2007/053386

(56) References cited:
- WO-A-2004/037801
- WO-A-2007/035425
- RUSSELL R: "One-Pot Synthesis Aids Scale-Up and Data Collection" PHARMACEUTICAL TECHNOLOGY, vol. 27, 2003, page 17,22, XP002433225
- KJELL D P ET AL: "A NOVEL, NONAQUEOUS METHOD FOR REGENERATION OF ALDEHYDES FROM BISULFITE ADDUCTS" JOURNAL OF ORGANIC CHEMISTRY, AMERICAN CHEMICAL SOCIETY. EASTON, US, vol. 64, no. 15, 7 February 1999 (1999-02-07), pages 5722-5724, XP003013995 ISSN: 0022-3263

## Description

### FIELD OF THE INVENTION

The present invention relates to processes for the preparation of cyclopropylamine derivatives, useful for the treatment of disorders and conditions mediated by the histamine receptor.

### BACKGROUND OF THE INVENTION

US Patent Application Publication 2004-0110746 A1, published April 21, 2005 (also published as PCT Publication WO 04/037801, May 6, 2004) discloses novel piperazinyl and diazepanyl benzamide derivatives useful for the treatment of histamine receptor mediated disorders. More specifically, the compounds are useful for the treatment of disorders and conditions mediated by the H₃ receptor. More particularly, the compounds are useful for treating or preventing neurologic disorders including sleep/wake and arousal/vigilance disorders (e.g. insomnia and jet lag), attention deficit hyperactivity disorders (ADHD), learning and memory disorders, cognitive dysfunction, migraine, neurogenic inflammation, dementia, mild cognitive impairment (pre-dementia), Alzheimer's disease, epilepsy, narcolepsy, eating disorders, obesity, motion sickness, vertigo, schizophrenia, substance abuse, bipolar disorders, manic disorders and depression, as well as other histamine H₃ receptor mediated disorders such as upper airway allergic response, asthma, itch, nasal congestion and allergic rhinitis in a subject in need thereof. For example, methods for preventing, inhibiting the progression of, or treating upper airway allergic response, asthma, itch, nasal congestion and allergic rhinitis.

US Patent Application Publication 2004-0110746 A1, published April 21, 2005 (also published as PCT Publication WO 04/037801, May 6, 2004) discloses a process for the preparation of the piperazinyl and diazepanyl benzamides. There remains a need for processes for the preparation of piperazinyl and diazepanyl benzamide derivatives that are suitable for large scale / commercial applications.

### SUMMARY OF THE INVENTION

The present invention is further directed to a process for the preparation of compounds of formula (IIs) 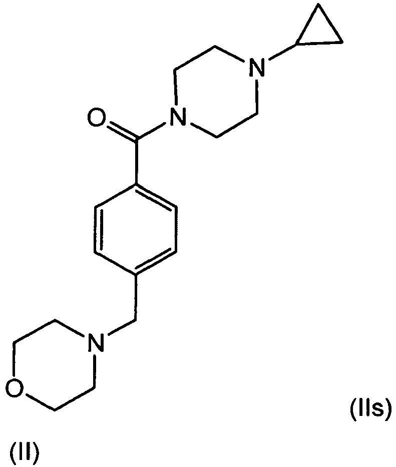 and hydrates, solvates, and pharmaceutically acceptable salts thereof;
comprising 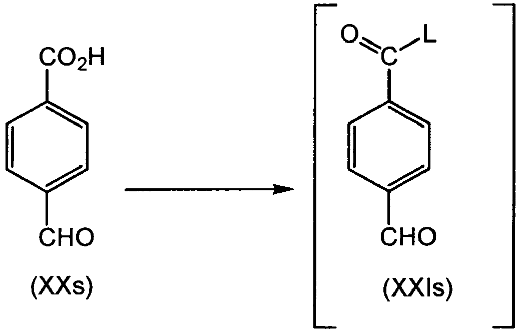 reacting a compound of formula (XXs); in a first organic solvent; to yield the corresponding compound of formula (XXIs), wherein L is a leaving group; and wherein the compound of formula (XXIs) is not isolated; 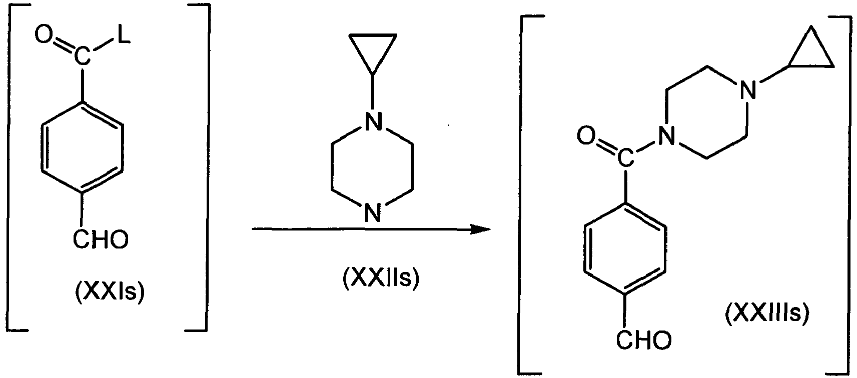 reacting the compound of formula (XXIs) with a compound of formula (XXIIs); in the presence of an organic or inorganic base; in a second organic solvent; to yield the corresponding compound of formula (XXIIIs); wherein the compound of formula (XXIIIs) is not isolated; 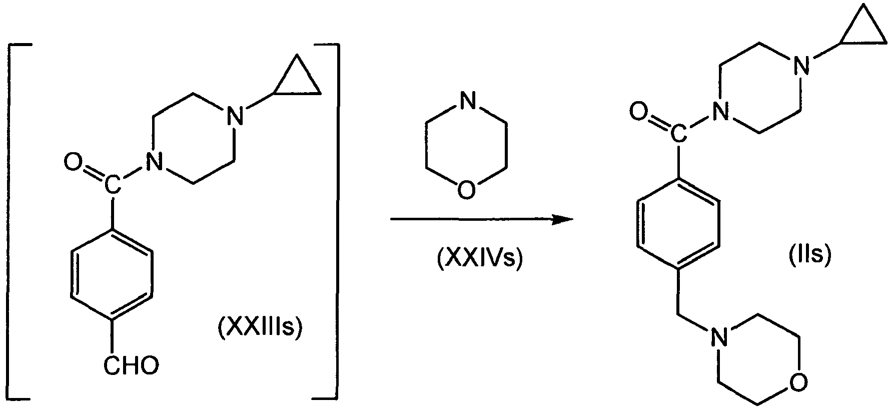 and reacting the compound of formula (XXIIIs) with a compound of formula (XXIVs); in the presence of a reducing agent; in a third organic solvent; to yield the corresponding compound of formula (IIs).

In an embodiment, the present invention is directed to processes for the preparation of a compound of formula (IIs) 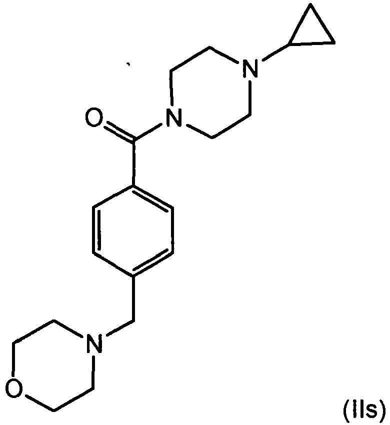 also known as (4-cyclopropyl-piperazin-1-yl)-(4-morpholin-4-ylmethyl-phenyl)-methanone, or its pharmaceutically acceptable salt thereof, preferably the di-hydrochloride salt.

Also disclosed herein is a pharmaceutical composition comprising a pharmaceutically acceptable carrier and a product prepared according to any of the processes described herein. Also disclosed herein is a pharmaceutical composition made by mixing a product prepared according to any of the processes described herein and a pharmaceutically acceptable carrier. Also disclosed herein is a process for making a pharmaceutical composition comprising mixing a product prepared according to any of the processes described herein and a pharmaceutically acceptable carrier.

Also disclosed herein are methods of treating a disorder mediated by histamine, preferably, the H₃ histamine receptor, (selected from the group consisting of neurologic disorders including sleep/wake and arousal/vigilance disorders (e.g. insomnia and jet lag), attention deficit hyperactivity disorders (ADHD), learning and memory disorders, cognitive dysfunction, migraine, neurogenic inflammation, dementia, mild cognitive impairment (pre-dementia), Alzheimer's disease, epilepsy, narcolepsy, eating disorders, obesity, motion sickness, vertigo, schizophrenia, substance abuse, bipolar disorders, manic disorders and depression, as well as other histamine H₃ receptor mediated disorders such as upper airway allergic response, asthma, itch, nasal congestion and allergic rhinitis) comprising administering to a subject in need thereof, a therapeutically effective amount of a products prepared according to any of the processes described herein or a pharmaceutical composition as described above.

Also disclosed herein is the use of a product prepared according to any of the processes described herein in the preparation of a medicament for treating: (a) a sleep/wake disorder, (b) an arousal/vigilance disorders, (c) insomnia, (d) jet lag, (e) attention deficit hyperactivity disorders (ADHD), (f) a learning disorder, (g) a memory disorder, (h) cognitive dysfunction, (i) migraine, (j) neurogenic inflammation, (k) dementia, (I) mild cognitive impairment (pre-dementia), (m) Alzheimer's disease, (n) epilepsy, (o) narcolepsy, (p) an eating disorder, (q) obesity, (r) motion sickness, (s) vertigo, (t) schizophrenia, (u) substance abuse, (v) bipolar disorder, (w) manic disorder, (x) depression, (y) upper airway allergic response, (z) asthma, (aa) itch, (bb) nasal congestion or (cc) allergic rhinitis, in a subject in need thereof.

### DETAILED DESCRIPTION OF THE INVENTION

Disclosed herein are processes for the preparation of compounds of formula (II) 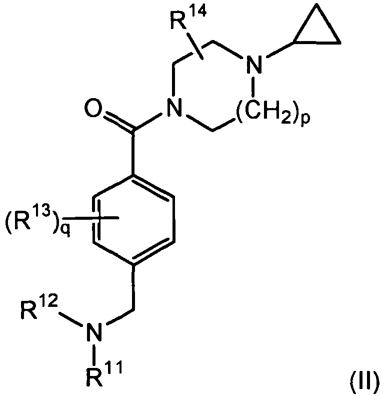 wherein p, R¹⁴, q, R¹³, R¹¹ and R¹² are as herein defined, useful for the treatment of disorders and conditions modulated by a histamine receptor.

As used herein, the terms "**including**", "**containing**" and "**comprising**" are used herein in their open, non-limiting sense.

The term "**alkyl**" refers to a straight- or branched-chain alkyl group having from 1 to 12 carbon atoms in the chain. Exemplary alkyl groups include methyl (Me, which also may be structurally depicted by "*I*"), ethyl (Et), n-propyl, isopropyl, butyl, isobutyl, sec-butyl, tert-butyl (tBu), pentyl, isopentyl, tert-pentyl, hexyl, isohexyl, and the like.

The term "**alkylene**" refers to a divalent straight- or branched-chain alkyl group having from 1 to 12 carbon atoms in the chain. Exemplary alkylene groups include methylene, ethylene, propylene, and the like.

The term "alkenyl" refers to a straight- or branched-chain alkenyl group having from 2 to 12 carbon atoms in the chain. (The double bond of the alkenyl group is formed by two sp² hybridized carbon atoms.) Illustrative alkenyl groups include prop-2-enyl, but-2-enyl, but-3-enyl, 2-methylprop-2-enyl, hex-2-enyl, and the like.

The term "alkynyl" refers to a straight- or branched-chain alkynyl group having from 2 to 12 carbon atoms in the chain. (The triple bond of the alkynyl group is formed by two sp hybridized carbon atoms.) Illustrative alkynyl groups include prop-2-ynyl, but-2-ynyl, but-3-ynyl, 2-methylbut-2-ynyl, hex-2-ynyl, and the like.

The term "aryl" refers to a monocyclic, or fused or spiro polycyclic, aromatic carbocycle (ring structure having ring atoms that are all carbon) having from 3 to 12 ring atoms per ring. (Carbon atoms in aryl groups are sp² hybridized.) Illustrative examples of aryl groups include phenyl, naphthyl, anthracenyl; phenanthrenyl, and the like.

The term "heteroaryl" refers to a monocyclic, or fused bicyclic or polycyclic, aromatic heterocycle (ring structure having ring atoms selected from carbon atoms as well as nitrogen, oxygen, and sulfur heteroatoms) having from 3 to 12 ring atoms per ring. Illustrative examples of heteroaryl groups include the following moieties: 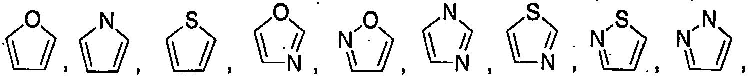 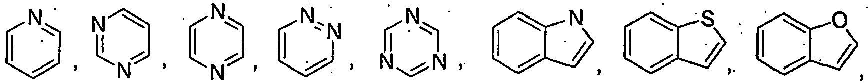 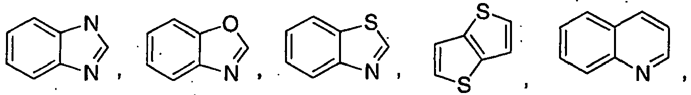 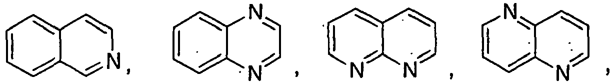 and the like.

The term "cycloalkyl" refers to a saturated or partially saturated, monocyclic or fused or spiro polycyclic, carbocycle having from 3 to 12 ring atoms per ring. Illustrative examples of cycloalkyl groups include the following moieties: 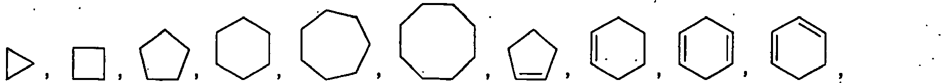 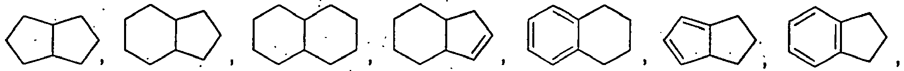 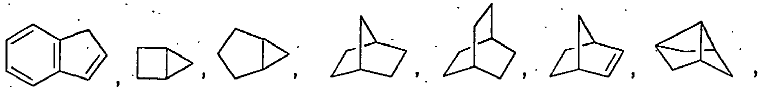 and the like.

A "heterocycloalkyl" refers to a monocyclic, or fused or spiro polycyclic, ring structure that is saturated or partially saturated and has from 3 to 12 ring atoms per ring selected from C atoms and N, O, and S heteroatoms. Illustrative examples of heterocycloalkyl groups include: 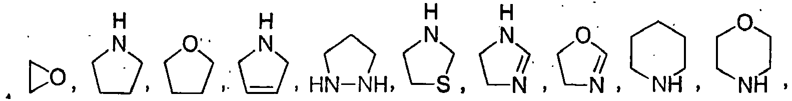 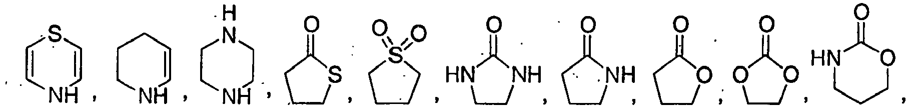 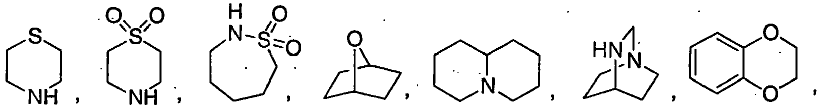 and
the like.

The term "**halogen**" represents chlorine, fluorine, bromine or iodine. The term "halo" represents chloro, fluoro, bromo or iodo.

The term "**substituted**" means that the specified group or moiety bears one or more substituents. The term "**unsubstituted**" means that the specified group bears no substituents. The term "**optionally substituted**" means that the specified group is unsubstituted or substituted by one or more substituents.

Where the term "substituted" is used to describe a structural system, the substitution is meant to occur at any valency-allowed position on the system.

Abbreviations used in the specification, particularly in the Schemes and Examples, are as follows:

| | | |
|---|---|---|
| CDI | = | N,N'-Carbonyldiimidazole |
| DCM | = | Dichloromethane |
| DIPEA | = | Diisopropyl ethyl amine |
| DMF | = | Dimethylformamide |
| DSC | | Differential Scanning Calorimetry |
| DVS | = | Dynamic Vapour Sorption |
| EDCI | = | 1-(3-Dimethylaminopropyl)-3-ethylcarbodiimide hydrochloride |
| Et₂O | = | Diethyl Ether |
| EtOAc | = | Ethyl Acetate |
| EtOH | = | Ethanol |
| HOBt | = | 1-Hydroxybenzotriazole |
| HPLC | = | High Performance Liquid Chromatography |
| MeOH | = | Methanol |
| MTBE | = | Methyl t-Butyl Ether |
| NaBH(OAC)₃ | = | Sodium triacetoxyborohydride |
| NMR | = | Nuclear Magnetic Resonance |
| OBt | = | -O-(1-benzotriazolyl) |
| RH | = | Relative Humidity |
| TEA or Et₃N | = | Triethylamine |
| THF | = | Tetrahydrofuran |
| TLC | = | Thin Layer Chromatography |
| XRD | | X-Ray Diffraction |

With reference to substituents, the term "**independently**" means that when more than one of such substituents is possible, such substituents may be the same or different from each other.

The term "**subject**" as used herein, refers to an animal, preferably a mammal, most preferably a human, who has been the object of treatment, observation or experiment.

The term "**therapeutically effective amount**" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term "**composition**" is intended to encompass a product comprising the specified ingredients in the specified amounts, as well as any product which results, directly or indirectly, from combinations of the specified ingredients in the specified amounts.

To provide a more concise description, some of the quantitative expressions given herein are not qualified with the term "**about**". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

As used herein, unless otherwise noted, the term "**leaving group**" shall mean a charged or uncharged atom or group which departs during a substitution or displacement reaction. Suitable examples include, but are not limited to, Br, Cl, imidazolyl, and the like.

Where the compounds prepared by processes according to this invention have at least one **chiral center**, they may accordingly exist as enantiomers. Where the compounds possess two or more chiral centers, they may additionally exist as diastereomers. It is to be understood that all such isomers and mixtures thereof are encompassed within the scope of the present disclosure. Preferably, wherein the compound is present as an enantiomer, the enantiomer is present at an enantiomeric excess of greater than or equal to about 80%, more preferably, at an enantiomeric excess of greater than or equal to about 90%, more preferably still, at an enantiomeric excess of greater than or equal to about 95%, more preferably still, at an enantiomeric excess of greater than or equal to about 98%, most preferably, at an enantiomeric excess of greater than or equal to about 99%. Similarly, wherein the compound is present as a diastereomer, the diastereomer is present at an diastereomeric excess of greater than or equal to about 80%, more preferably, at an diastereomeric excess of greater than or equal to about 90%, more preferably still, at an diastereomeric excess of greater than or equal to about 95%, more preferably still, at an diastereomeric excess of greater than or equal to about 98%, most preferably, at an diastereomeric excess of greater than or equal to about 99%.

Furthermore, some of the crystalline forms for the compounds prepared by processes of the present invention may exist as polymorphs and as such are intended to be included in the present invention. In addition, some of the compounds prepared by processes of the present invention may form solvates with water (i.e., hydrates) or common organic solvents.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art. For example, one skilled in the art will recognize that in the processes of the present invention, it may be necessary and /or desirable to protect substituent groups such as (C₁₋₈alkylcarbonyl)C₁₋₈alkyl.

Also disclosed herein are **prodrugs** of the compounds prepared by processes of this invention. In general, such prodrugs will be functional derivatives of the compounds which are readily convertible *in vivo* into the required compound. Thus, in the methods of treatment disclosed herein, the term "administering" shall encompass the treatment of the various disorders described with the compound specifically disclosed or with a compound which may not be specifically disclosed, but which converts to the specified compound *in vivo* after administration to the patient. Conventional procedures for the selection and preparation of suitable prodrug derivatives are described, for example, in "Design of Prodrugs", ed. H. Bundgaard, Elsevier, 1985.

For use in medicine, the salts of the compounds prepared by the processes of this invention refer to non-toxic "**pharmaceutically acceptable salts**." Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds prepared by processes of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:
acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:
acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1 S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Also disclosed herein are processes for the preparation of compounds of formula (II), or an enantiomer, diastereomer, hydrate, solvate thereof, or a pharmaceutically acceptable salt, amide or ester thereof, wherein R¹¹, R¹², R¹³, R¹⁴, p, and q have any of the meanings defined hereinabove and equivalents thereof, or at least one of the following assignments and equivalents thereof. Such assignments may be used where appropriate with any of the definitions, claims or embodiments defined herein:

Also disclosed herein are processes for the preparation of compounds of formula (II) wherein p is 1.

Also disclosed herein are to processes for the preparation of compounds of formula (II) wherein R¹¹ and R¹² are each independently selected from the group consisting of methyl, ethyl, propyl, isopropyl, butyl, isobutyl, tert-butyl, cyclopropyl, cyclobutyl, cyclopentyl, cyclohexyl, azetidinyl, pyrrolidinyl, piperidinyl, morpholinyl, and thiomorpholinyl, each optionally substituted as described above.

Also disclosed herein are processes for the preparation of compounds of formula (II) wherein R¹¹ and R¹² taken together with the nitrogen to which they are attached form 2-methylpyrrolidinyl, 3-hydroxypyrrolidinyl, 3-dimethylaminopyrrolidinyl, 2,5-dimethylpyrrolidinyl, 2-trifluoromethylpyrrolidinyl, 2-hydroxymethylpyrrolidinyl, piperidinyl, 4-fluoropiperidinyl, 3,3-difluoropiperidinyl, 4,4-difluoropiperidinyl, 3-trifluoromethylpiperidinyl, 4-trifluoromethylpiperidinyl, morpholinyl, 3-hydroxypiperidinyl, 4-hydroxypiperidinyl, 2-hydroxymethylpiperidinyl, 3-hydroxymethylpiperidinyl, 4-hydroxymethylpiperidinyl, 4-hydroxyethylpiperidinyl, 3-methylmorpholin-4-yl, 3-hydroxymethylmorpholin-4-yl, 2-hydroxymethylmorpholin-4-yl, 2,6-dimethylmorpholin-4-yl, thiomorpholinyl, 1,1-dioxo-thiomorpholin-4-yl, or 2-methylmorpholin-4-yl.

Also disclosed herein are processes for the preparation of compounds of formula (II) wherein, R¹¹ and R¹² taken together with the nitrogen to which they are attached form piperidinyl, 4-fluoropiperidinyl, 4,4-difluoropiperidinyl, morpholinyl, or 3-methylmorpholin-4-yl. In another embodiment, the present invention is directed to processes for the preparation of compounds of formula (II) wherein R¹¹ and R¹² taken together with the nitrogen to which they are attached form morpholinyl.

Also disclosed herein are processes for the preparation of compounds of formula (II) wherein each R¹³ is independently selected from the group consisting of methyl, methoxy, and fluoro.

Also disclosed herein are processes for the preparation of compounds of formula (II) wherein q is 0.

Also disclosed herein are processes for the preparation of compounds of formula (II) wherein **R¹⁴** is -H or methyl.

Also disclosed herein are processes for the preparation of compounds of formula (II) that satisfy any one of the combinations of definitions given herein and equivalents thereof.

The present invention is directed to processes for the preparation of compounds of formula (II) selected from the group consisting of (4-cyclopropyl-piperazin-1-yl)-(4-morpholin-4-ylmethyl-phenyl)-methanone and (4-cyclopropyl-piperazin-1-yl)-(4-morpholin-4-ylmethyl-phenyl)-methanone dihydrochloride.

The compounds prepared by the processes of the present invention are modulators of the histamine H₃ receptor, and as such, the compounds are useful in the treatment of disease states in which the histamine H₃ receptor is involved. Particularly, the compounds may be used in methods for treating or preventing neurologic or neuropsychiatric disorders including sleep/wake and arousal/vigilance disorders (e.g. insomnia and jet lag), attention deficit hyperactivity disorders (ADHD), learning and memory disorders, cognitive dysfunction, migraine, neurogenic inflammation, dementia, mild cognitive impairment (pre-dementia), Alzheimer's disease, epilepsy, narcolepsy with or without associated cataplexy, cataplexy, disorders of sleep/wake homeostasis, idiopathic somnolence, excessive daytime sleepiness (EDS), circadian rhythm disorders, sleep/fatigue disorders, fatigue, drowsiness associated with sleep apnea, sleep impairment due to perimenopausal hormonal shifts, Parkinson's-related fatigue, MS-related fatigue, depression-related fatigue, chemotherapy-induced fatigue, eating disorders, obesity, motion sickness, vertigo, schizophrenia, substance abuse, bipolar disorders, manic disorders and depression, as well as other disorders in which the histamine H₃ receptor is involved, such as upper airway allergic response, asthma, itch, nasal congestion and allergic rhinitis in a subject in need thereof. For example, the invention features processes for preparing compounds for use in methods for preventing, inhibiting the progression of, or treating upper airway allergic response, asthma, itch, nasal congestion and allergic rhinitis. Excessive daytime sleepiness (EDS) may occur with or without associated sleep apnea, shift work, fibromyalgia, MS, and the like.

The present invention is directed to a process for the preparation of a compound of formula (IIs). The process of the present invention is advantageous for large scale and / or commercial purposes because it does not require isolation and / or purification of oily intermediates; and does not require column chromatography which is impractical and highly cost prohibitive on a large and / or commercial scale. Additionally, the process of the present invention may be completed in a single solvent system, whereas a similar process, disclosed in US Patent Application Publication 2004-0010746 A1, published April 21, 2005 (also published as PCT Publication WO 2004/037801, May 6, 2004) would requires multiple solvents (including reaction and extractive work-up solvents) if applied to the compounds of formula (II).

Disclosed herein is a process for the preparation of compounds of formula (II), as described in more detail in Scheme 1, below.

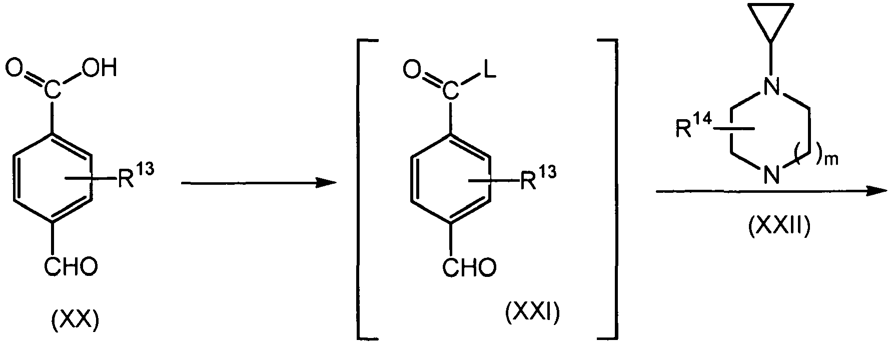

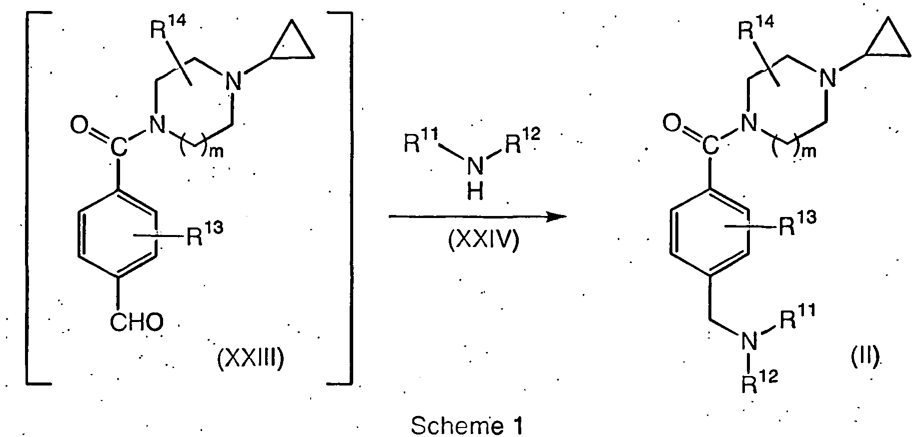

Accordingly, a suitably substituted compound of formula (XX), a known compound or compound prepared by known methods, is activated according to known methods, in a first organic solvent; to yield the corresponding compound of formula (XXI), wherein L is a suitable leaving group such as chloro, bromo, - OC(O)O-C₁₋₄alkyl, OBt (wherein the activating agent is HOBt), -imidazolide (wherein the activating agent is CDI), and the like; preferably chloro.

For example, wherein L is chloro, the compound of formula (XX) is reacted with a suitable chlorinating agent such,as oxalyl chloride, thionyl chloride, phosphorus oxychloride, and the like, preferably about 1.05 equivalents of oxalyl chloride in the presence of a catalytic amount of DMF; in an organic solvent such as THF, toluene, dichloromethane, dichloroethane, acetonitrile, and the like, preferably THF. Alternatively, the compound of formula (XX) is reacted with Vilsmeier's reagent (chloromethylene-dimethyl-ammonium chloride) in an organic solvent such as DCM; at a temperature in the range of from about 0°C to about room temperature.

The compound of formula (XXI) is not isolated.

The compound of formula (XXI) is reacted with a suitably substituted compound of formula (XXII), a known compound or compound prepared by known methods, wherein the compound of formula (XXII) is preferably present in an amount equal to about one equivalent, more preferably about 0.95 equivalents; in the presence of an organic or inorganic base (solid or aqueous)
such as TEA, DIPEA, pyridine, NaOH, KOH, sodium carbonate, potassium carbonate, and the like, preferably 50% aqueous NaOH; wherein the base is organic, preferably in the absence of water; in a second organic solvent such as THF, toluene, acetonitrile, and the like, preferably THF; to yield the corresponding compound of formula (XXIII).

The compound of formula (XXIII) is not isolated.

The compound of formula (XXIII) is reacted with a suitably substituted compound of formula (XXIV), a known compound or compound prepared by known methods, wherein the compound of formula (XXIV) is preferably present in an amount greater than about one equivalent, more preferably in an amount in the range of from about 1 to about 5 equivalents, more preferably still in an amount in the range of from about 1.5 to about 2.5 equivalents, most preferably in an amount in the range of from about 1.5 to about 2 equivalents; in the presence of a reducing agent such as NaBH(OAc)₃, NaBH₄, sodium cyanoborohydride, and the like, preferably, NaBH(OAc)₃; wherein the reducing agent is preferably present in an amount in the range of from about 1 to about 2 equivalents, more preferably in an amount in the range of from about 1.25 to about 1.5 equivalents; in a third organic solvent such as THF, toluene, acetonitrile, and the like, preferably, THF; to yield the corresponding compound of formula (II).

The compound of formula (II) is further, optionally isolated and / or purified according to known methods. Alternatively, the compound of formula (II) is not isolated and / or purified, rather, the compound of formula (II) is reacted according to known methods, to yield a corresponding pharmaceutically acceptable salt of the compound of formula (II).

Preferably, the first organic solvent, the second organic solvent and the third organic solvent are the same. Preferably, the conversion of the compound of formula (XX) to the corresponding compound of formula (II) is completed in a single solvent system.

According to the disclosure, the compound of formula (II) is further reacted with a suitably selected pharmaceutically acceptable acid to yield the corresponding pharmaceutically acceptable salt of the compound of formula (II). In an embodiment of the present invention, the compound of formula (II) is not isolated and is reacted with a suitably selected pharmaceutically acceptable acid to yield the corresponding pharmaceutically acceptable salt of the compound of formula (II).

The present invention is directed to a process for the preparation of the compound of formula (IIs), also known as also known as (4-cyclopropyl-piperazin-1-yl)-(4-morpholin-4-ylmethyl-phenyl)-methanone, as described in more detail in Scheme 2, below.

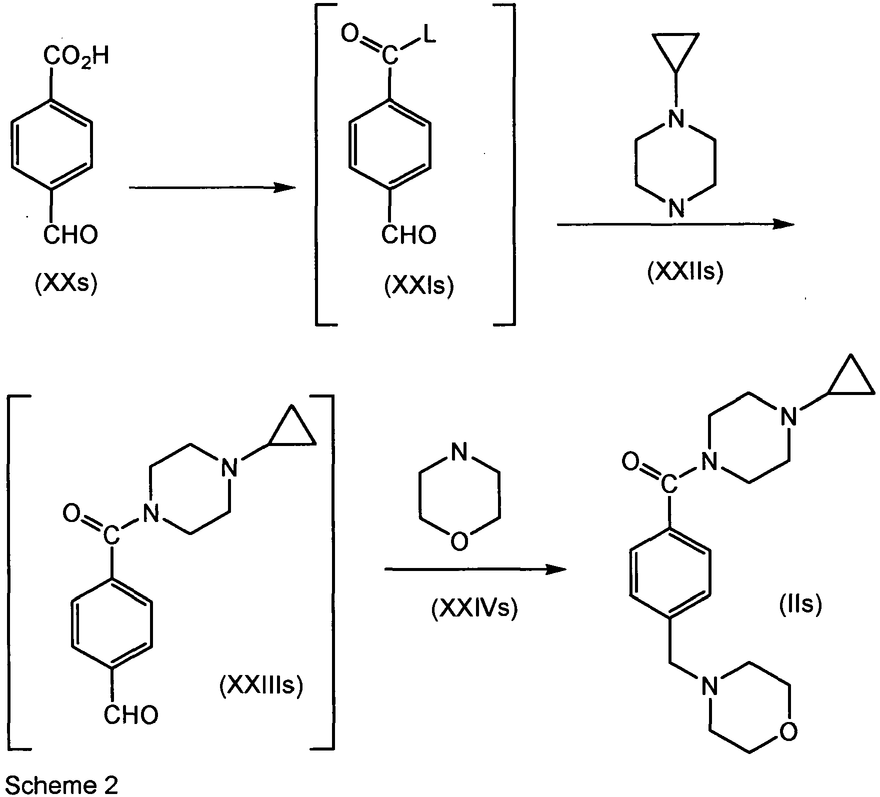

Accordingly, a suitably substituted compound of formula (XXs), also known as 4-formyl-benzaldehyde, a known compound, is activated according to known methods, in a first organic solvent, to yield the corresponding compound of formula (XXIs), wherein L is chloro.

For example, the compound of formula (XXs) is reacted with a suitable chlorinating agent such as oxalyl chloride, thionyl chloride, phosphorus oxychloride, and the like, preferably about 1.05 equivalents of oxalyl chloride in the presence of a catalytic amount of DMF; in an organic solvent such as THF, toluene, dichloromethane, dichloroethane, acetonitrile, and the like, preferably THF. Alternatively, the compound of formula (XXs) is reacted with Vilsmeier's reagent (chloromethylene-dimethyl-ammonium chloride) in an organic solvent such as DCM; at a temperature in the range of from about 0°C to about room temperature.

The compound of formula (XXIs) is not isolated.

The compound of formula (XXIs) is reacted with a suitably substituted compound of formula (XXIIs), also known as N-cyclopropyl-piperazine, a known compound, wherein the compound of formula (XXIIs) is preferably present in an amount equal to about one equivalent, more preferably about 0.95 equivalents; in the presence of an organic or inorganic base (solid or aqueous) such as TEA, DIPEA, pyridine, NaOH, KOH, sodium carbonate, potassium carbonate, and the like, preferably 50% aqueous NaOH; wherein the base is organic, preferably in the absence of water; in a second organic solvent such as THF, toluene, acetonitrile, and the like, preferably THF; to yield the corresponding compound of formula (XXIIIs), also known as 4-(4-isopropyl-piperazine-1-carbonyl)-benzaldehyde.

The compound of formula (XXIIIs) is not isolated.

The compound of formula (XXIIIs) is reacted with a suitably substituted compound of formula (XXIVs), also known as morpholine, a known compound, wherein the compound of formula (XXIVs) is preferably present in an amount greater than about one equivalent, more preferably in an amount in the range of from about 1 to about 5 equivalents, more preferably still in an amount in the range of from about 1.5 to about 2.5 equivalents, most preferably in an amount in the range of from about 1.5 to about 2 equivalents; in the presence of a reducing agent such as NaBH(OAc)₃, NaBH₄, sodium cyanoborohydride, and the like, preferably, NaBH(OAc)₃; wherein the reducing agent is preferably present in an amount in the range of from about 1 to about 2 equivalents, more preferably in an amount in the range of from about 1.25 to about 1.5 equivalents; in a third organic solvent such as THF, toluene, acetonitrile, and the like, preferably, THF; to yield the corresponding compound of formula (IIs), also known as (4-cyclopropyl-piperazin-1-yl)-(4-morpholin-4-ylmethyl-phenyl)-methanone.

The compound of formula (IIs) is further, optionally isolated and / or purified according to known methods. Alternatively, the compound of formula (IIs) is not isolated and / or purified, rather, the compound of formula (IIs) is reacted according to known methods, to yield a corresponding pharmaceutically acceptable salt of the compound of formula (IIs).

The first organic solvent, the second organic solvent and the third organic solvent are the same

In an embodiment of the present invention, the compound of formula (IIs) is further reacted with a suitably selected pharmaceutically acceptable acid to yield the corresponding pharmaceutically acceptable salt of the compound of formula (IIs). In an embodiment of the present invention, the compound of formula (IIs) is not isolated and is reacted with a suitably selected pharmaceutically acceptable acid to yield the corresponding pharmaceutically acceptable salt of the compound of formula (IIs).

Preferably, the compound of formula (IIs) is isolated according to known methods, for example by solvent evaporation. The compound of formula (IIs) may be further, optionally, reacted according to known methods, to yield its corresponding pharmaceutically acceptable salt, preferably its corresponding di-hydrochloride salt.

The compounds or compositions prepared by the processes of the invention may be formulated and administered to a subject by any conventional route of administration, including, but not limited to, intravenous, oral, subcutaneous, intramuscular, intradermal and parenteral administration. The quantity of the compound which is effective for treating each condition may vary, and can be determined by one of ordinary skill in the art.

Also disclosed herein are pharmaceutical compositions comprising one or more compounds prepared by the processes of this invention in association with a pharmaceutically acceptable carrier and optionally additional pharmaceutical agents such as H₁ antagonists or SSRIs (Selective Serotonin Reuptake Inhibitors). Preferably these compositions are in unit dosage forms such as pills, tablets, caplets, capsules (each including immediate release, timed release and sustained release formulations), powders, granules, sterile parenteral solutions or suspensions (including syrups and emulsions), metered aerosol or liquid sprays, drops, ampoules, autoinjector devices or suppositories; for oral, parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid pre-formulation composition containing a homogeneous mixture of a compound prepared by the processes of the present invention, or a pharmaceutically acceptable salt thereof. When referring to these pre-formulation compositions as homogeneous, it is meant that the active ingredient is dispersed evenly throughout the composition so that the composition may be readily subdivided into equally effective dosage forms such as tablets, pills and capsules. This solid pre-formulation composition is then subdivided into unit dosage forms of the type described above containing from 5 to about 1000 mg of the active ingredient prepared by the processes of the present invention. Examples include 5 mg, 7 mg, 10 mg, 15 mg, 20 mg, 35 mg, 50 mg, 75 mg, 100 mg, 120 mg, 150 mg, and so on. The tablets or pills of the disclosed compositions can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer, which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the compounds and compositions prepared by the processes of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

Advantageously, compounds prepared by the processes of the present invention may be administered in a single daily dose, or the total daily dosage may be administered in divided doses of two, three or four times daily. Furthermore, compounds prepared by the processes of the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The compound prepared by the processes of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phophatidylcholines.

Compounds prepared by the processes of the present invention may also be delivered by the use of monoclonal antibodies as individual carriers to which the compound molecules are coupled. The compounds prepared by the processes of the present invention may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palmitoyl residue. Furthermore, the compounds prepared by the processes of the present invention may be coupled to a class of biodegradable polymers useful in achieving controlled release of a drug, for example, polylactic acid, polyepsilon caprolactone, polyhydroxy butyric acid, polyoesters, polyacetals, polydihydropyrans, polycyanoacrylates and cross-linked or amphipathic block copolymers of hydrogels.

Compounds prepared by the processes of this invention may be administered in any of the foregoing compositions and according to dosage regimens established in the art whenever treatment is required.

The daily dosage of the products may be varied over a wide range from 1 to 1,000 mg per adult human per day. For oral administration, the compositions are preferably provided in the form of tablets containing 1.0, 5.0, 10.0, 15.0, 25.0, 50.0, 100, 250 and 500 milligrams of the active ingredient for the symptomatic adjustment of the dosage to the subject to be treated. An effective amount of the drug is ordinarily supplied at a dosage level of from about 0.01 mg/kg to about 20 mg/kg of body weight per day. Preferably, the range is from about 0.02 mg/kg to about 10 mg/kg of body weight per day, and especially from about 0.05 mg/kg to about 10 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

The following Examples are set forth to aid in the understanding of the invention, and are not intended and should not be construed to limit in any way the invention set forth in the claims which follow thereafter.

In the Examples which follow, some synthesis products are listed as having been isolated as a residue. It will be understood by one of ordinary skill in the art that the term "residue" does not limit the physical state in which the product was isolated and may include, for example, a solid, an oil, a foam, a gum, a syrup, and the like.

### Example1

### 4-Formyl-benzoyl chloride

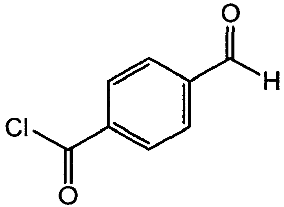

To a thin suspension of 4-carboxybenzaldehyde (600g, 3.92 mol) in tetrahydrofuran (2664g, 36.57 mol) was added dimethylformadehyde (11.48g, 0.16mol) and the reaction mixture was cooled to 0-5°C with an ice bath. The reaction mixture was then stirred at 0°C while oxalyl chloride (608.69g, 4.70 mol) was added slowly. The reaction mixture was stirred until it was deemed complete by ¹HNMR to yield the title compound. The reaction mixture was used in the next step without further manipulation.

¹HNMR (CDCl3): 10.15 (s, 1H), 8.35 (d, 2H), 8.05 (d, 2H)

### Example 2 (reference example)

### 4-(4-Isopropyl-piperazine-1-carbonyl)-benzaldehyde

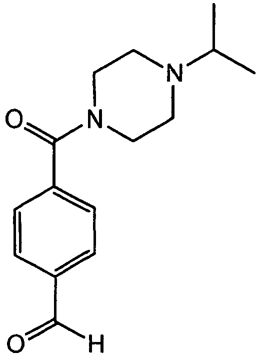

A solution of 4-formyl-benzoyl chloride (2.80, 16.65 mol) (prepared as in Example 1 above) in toluene (43.3g, 469.39 mmol) was added slowly to a solution of NaHCO₃ (0.8g, 9.52 mmol) and 4-isopropylpiperazine (2.50g, 18.35mmol) in water (5g, 277 mmol) at 0°C. The reaction mixture was vigorously stirred until the reaction was deemed complete. The layers were split and the toluene phase was concentrated to yield the title compound as a yellow oil.

¹HNMR (CDCl₃): 10.15 (s, 1H), 7.95 (d, 2H), 7.55 (d, 2H), 3.75 (br s, 2H), 3.40(br s, 2H), 2.75 (m, 1H), 2.55 (br s, 2H), 2.41 (br s, 2H), 1.09 (d, 6H)

### Example 3

### 4-(4-Isopropyl-piperazine-1-carbonyl)-benzaldehyde

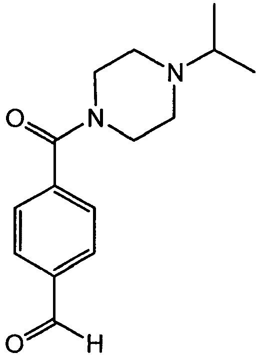

4-Isopropyl-piperazine (79.53g, 0.620mol), THF (444g, 5.04 mol), water (36g, 2 mol) and a 50% solution of sodium hydroxide (130.6g, 1.63mol) were charged to a reaction vessel and cooled to 0-5 °C. 4-Formyl-benzoyl chloride in THF (110.08g, 0.630 mol) was added to the 4-isopropyl-piperazine reaction mixture while maintaining the temperature below about 10°C. The resulting white suspension was stirred at room temperature until the reaction was deemed complete. Water was added to the reaction slurry and the resulting hazy solution was filtered over Celite to remove insolubles. The filtered reaction solution was settled and the water layer was removed. The product /THF layer was dried sequentially with magnesium sulfate and molecular sieves. The product solution (KF ≤ 0.5%) was stored at 5°C for use without further manipulations.

### Example 4

### (4-Isopropyl-piperazin-1-yl)-(4-morpholin-4-ylmethyl-phenyl)-methanone

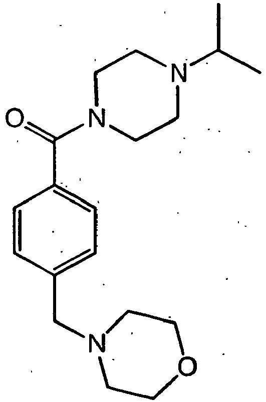

To a solution of 4-(4-isopropyl-piperazine-1-carbonyl)-benzaldehyde (4.0 g, 15.38 mmol) in THF (40 mL) was added morpholine (2.9 g, 33.83 mmol), and the resulting mixture was stirred at room temperature for 1 h before it was cooled to 0°C with an ice bath. The reaction mixture was then treated with NaBH(OAc)₃ (4.56 g, 21.53 mmol) in portions over 15 min. The resulting suspension was stirred at room temperature until it was deemed complete by HPLC. After completion, 10% NaOH (25mL) was added and the reaction was vigorously agitated for 15 min. The phases were separated and the aqueous layer was extracted with THF (20 mL). The organic layers were combined, dried (MgSO₄), filtered and concentrated to yield the title compound as a yellow oil.

¹H NMR (CDCl₃): 7.36 (s, 4H), 3.79 (br s, 2H), 3.71 (t, 4H), 3.51 (s, 2H), 3.44 (br s, 2H), 2.76-2.69 (m, 1H), 2.59 (br s, 2H), 2.44 (t, 6H), 1.05 (d, 6H).

### Example 5

### (4-Isopropyl-piperazin-1-yl)-(4-morpholin-4-ylmethyl-phenyl)-methanone

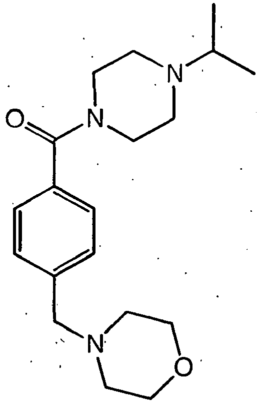

A THF solution of 1-(4-formylbenzoyl)-4-isopropylpiperazine (containing 945g of 1-(4-formylbenzoyl)-4-isopropylpiperazine and 3879 g of THF) was charged to a reaction vessel followed by the addition of morpholine (576.3g, 6.55 mol). After 20 min, the reaction was cooled to about 0-10°C and sodium triacetoxyborohydride (1167.3g, 5.23 mol) was added in portions. Upon reaction completion, 10% sodium hydroxide solution (3623.2 mL, 9.06 mol) was added slowly and the reaction mixture was stirred for 20 min. The layers were separated, and the aqueous layer was washed with THF. The combined organic layers were dried over magnesium sulfate. The dried THF solution of (4-Isopropyl-piperazin-1-yl)-(4-morpholin-4-ylmethyl-phenyl)-methanone was used without further manipulations.

### Example 6

### (4-Isopropyl-piperazin-1-yl)-(4-morpholin-4-ylmethyl-phenyl)-methanone Mono-succinate Salt

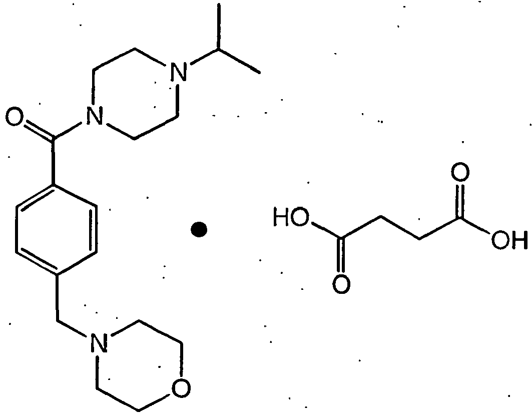

A THF solution (278.0g) of crude (4-isopropyl-piperazin-1-yl)-(4-morpholin-4-ylmethyl-phenyl)-methanone (59.4g, 0.179 mol) was heated to 40°C and succinic acid (27.53g, 0.233 mol) was added. The reaction mixture was heated to 60°C and filtered into a clean flask. The resulting solution was re-heated to 60°C and then cooled slowly, first to room temperature and then to -7°C. The resulting suspension was held at -7°C and filtered. The filter cake was washed with THF (60 mL) and the solid was dried overnight at 50°C under full vacuum to yield crude mono-succinate salt as a white solid.

A suspension of the crude mono-succinate salt (701.3g, 1.56 mol) in ethanol (7.01 L) was heated to 60-65°C. Any insoluble material was removed by filtration. The resulting clear solution was cooled slowly to -7°C. The slurry was filtered and washed with ethanol (700 mL). The filter cake was dried overnight at 50°C under full vacuum to yield the mono-succinate salt as a white crystalline solid.

| | | |
|---|---|---|
| M.P.: | 154-156°C | |
| Elemental Analysis For C₁₉H₂₉N₃O₂ x C₄H₆O₂: | | |
| | Calculated: | C, 61.45; H, 7.85; N, 9.35; H₂O, <0.1% |
| | Found: | C, 61.42; H, 7.84; N, 9.29; H₂O, <0.1% |
| MS: | [M + H]⁺ = 332; [2M + H]⁺ = 685. | |

### Example 7

### (4-Isopropyl-piperazin-1-yl)-(4-morpholin-4-ylmethyl-phenyl)-methanone Mono-fumarate Salt

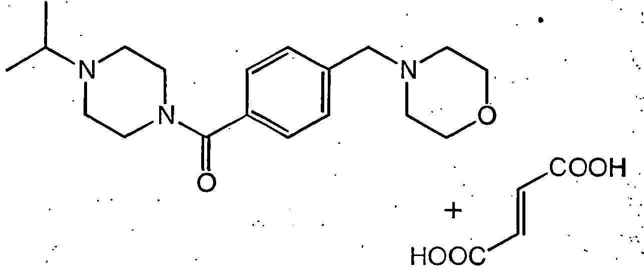

To a THF solution (40mL) of (4-isopropyl-piperazin-1-yl)-(4-morpholin-4-ylmethyl-phenyl)-methanone (3.0 g, 9.0 mmol) were added THF (40 mL) and fumaric acid (3.3 g, 28.4mmol). The resulting mixture was heated to 60°C and stirred for 0.5h. The resulting suspension was cooled to 0°C and the resulting precipitate was collected by filtration, washed with THF (20 mL), and dried in a vacuum oven at 65°C for 20 h to yield crude title compound as a white solid.

A suspension of crude (4-isopropyl-piperazin-1-yl)-(4-morpholin-4-ylmethyl-phenyl)-methanone, mono-fumarate (5.7g, 12.7mmol) in absolute EtOH (110 mL) was heated to 70°C. Any insoluble material was removed by filtration through a Celite pad. The filtrate was reheated to 65°C and then cooled to 0°C. The precipitate was collected by filtration and washed with MTBE (20 mL). The solids were dried in a vacuum oven at 65°C for 20 h to yield the title compound as a white solid.

| | | |
|---|---|---|
| M.P.: | 196-198°C. | |
| Elemental Analysis for C₁₉H₂₉N₃O₂ x C₄H₄O₄: | | |
| | Calculated: | C, 61.73; H, 7.43; N, 9.39 |
| | Found: | C, 61.44; H, 7.50; N, 9.30 |

### Example 8

### (4-Isopropyl-piperazin-1-yl)-(4-morpholin-4-ylmethyl-phenyl)-methanone, dihydrochloride monohydrate salt

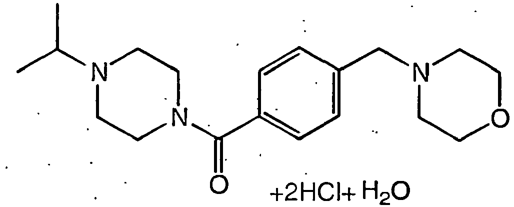

A solution of (4-isopropyl-piperazin-1-yl)-(4-morpholin-4-ylmethyl-phenyl)-methanone (2.0 g, 6.0 mmol) in absolute EtOH (20 mL) was treated with HCl_{(g)} (0.5g, 13.7mmol) at room temperature. The resulting suspension was stirred for 1 h, and then MTBE (5 mL) was added. The suspension was cooled to 0°C and filtered. The filter cake was washed with MTBE (20 mL), and the solid was dried in a vacuum oven at 60°C for 20 h to yield crude title compound as a white solid.

A suspension of crude (4-isopropyl-piperazin-1-yl)-(4-morpholin-4-ylmethyl-phenyl)-methanone, dihydrochloride (2.1 g, 5.2mmol) in absolute EtOH (30 mL) was heated to 78°C and H₂O (2.2mL) was added. The resulting solution was cooled to room temperature and MTBE (5mL) was added. The resulting suspension was cooled to 0°C and filtered. The filter cake was washed with MeOH (15 mL). The solids were dried in a vacuum oven at 105°C for 20 h to yield the title compound as a white solid.

| | | |
|---|---|---|
| M.P.: | decomp >220°C | |
| Elemental Analysis for C₁₉H₂₉N₃O₂ x 2HCl x H₂O: | | |
| | Calculated: | C, 53.97; H, 7.81; N, 9.94; Cl, 16.81; |
| | Found: | C, 54.13; H, 7.50; N, 9.90; Cl, 16.68; KF: 4.02% |

### Example 9

### (4-Isopropyl-piperazin-1-yl)-(4-morpholin-4-ylmethyl-phenyl)-methanone, dihydrobromide semi-hydrate salt

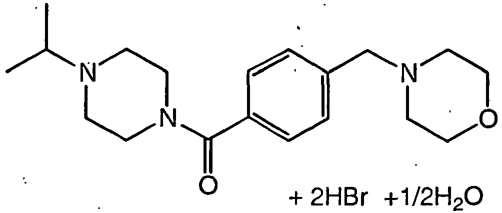

To a THF solution (40mL) of (4-isopropyl-piperazin-1-yl)-(4-morpholin--4-ylmethyl-phenyl)-methanone (3.0 g, 9.0 mmol) were added THF (40 mL) and 30% hydrogen bromide solution in acetic acid (3.7mL, 18.6mmol) while maintaining the temperature between 15°C and 20°C. The resulting suspension was stirred for 1 h, and then cooled to 0°C. The precipitate was collected by filtration, washed with THF (20 mL), and dried in a vacuum oven at 65°C for 20 h to yield crude title compound as a white solid.

A suspension of crude (4-isopropyl-piperazin-1-yl)-(4-morpholin-4-ylmethyl-phenyl)-methanone, dihydrobromide (4.9g, 9.9mmol) in MeOH (50 mL) was heated to 65°C. The resulting solution was cooled to 0°C and the precipitate was collected by filtration and washed with MeOH (15 mL). The solids were dried in a vacuum oven at 65°C for 20 h to yield the title compound as a white solid.

| | | |
|---|---|---|
| M.P.: | >290°C decomp | |
| Elemental Analysis for C₁₉H₂₉N₃O₂ x 2 HBr x 0.5H₂O: | | |
| | Calculated: | C, 45.39; H, 6.37; N, 8.36; Br, 31.85 |
| | Found: | C, 45.60; H, 6.32; N, 8.36; Br, 33.41 |
| | KF: | 2.02% |

### Example 10

### (4-Isopropyl-piperazin-1-yl)-(4-morpholin-4-ylmethyl-phenyl)-methanone Bis-maleate Salt

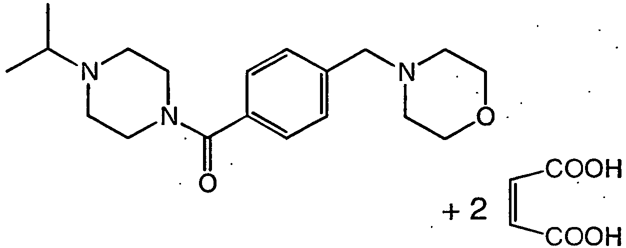

To a solution of (4-isopropyl-piperazin-1-yl)-(4-morpholin-4-ylmethyl-phenyl)-methanone (3.0 g, 9.05 mmol) in absolute EtOH (20 mL) was added, via an addition funnel, a solution of maleic acid (3.3 g, 19.8 mmol) in absolute EtOH (20 mL) over 10 min. The resulting suspension was stirred at room temperature for 15 min, at 75°C for 30 min, and was then allowed to cool to room temperature for 15 h. The reaction mixture was cooled further to 0°C and was then stirred for 2 h. The resulting precipitate was collected by suction filtration and washed with cold EtOH (20 mL). The wet solid was dried in a vacuum oven at 40°C for 6 h to yield the title compound as crude material, as a white solid.

A suspension of the crude (4-isopropyl-piperazin-1-yl)-(4-morpholin-4-ylmethyl-phenyl)-methanone, bis-maleate salt (3.0 g) in absolute EtOH (30 mL) was heated at 75°C for 1 h, and the resulting solution was filtered through a fine porosity glass frit. The filtrate was heated at 75°C and then cooled to room temperature over 2 h, with stirring, and Et₂O (10 mL) was added. The resulting suspension was cooled to 0°C for 2 h, the precipitate was collected by suction filtration and washed with Et₂O (20 mL) under nitrogen protection. The solids were dried in a vacuum oven at 45°C for 20 h to yield the title compound as a white crystalline solid.

| | | |
|---|---|---|
| MP: | 154.1 °C | |
| Elemental Analysis for C₂₇H₃₇N₃O₁₀: | | |
| | Calculated: | C, 57.54; H, 6.62; N, 7.46 |
| | Found: | C, 57.44; H, 6.66; N, 7.33. |

### Example 11

### Analysis Protocol for Compounds Prepared as in Examples 12-22

Hewlett Packard HPLC, Zorbax Eclipse XDB-C8, 5 uM, 4.6 x 150 mm column; Solvents used were H₂O/CH₃CN/0.05% Trifluoroacetic Acid; Gradient conditions were 1% - 99% CH₃CN gradient over 8 min, 99% CH₃CN for 2 min.

All reactions were carried out under a nitrogen atmosphere.

Mass spectra were obtained on an Agilent series 1100 MSD using electrospray ionization (ESI) in either positive or negative modes as indicated.

Thin-layer chromatography was performed using Merck silica gel 60 F₂₅₄ 2.5 cm x 7.5 cm 250 µm or 5.0 cm x 10.0 cm 250 µm pre-coated silica gel plates. Preparative thin-layer chromatography was performed using EM Science silica gel 60 F₂₅₄ 20 cm x 20 cm 0.5 mm pre-coated plates with a 20 cm x 4 cm concentrating zone.

NMR spectra were obtained on either a Bruker model DPX400 (400 MHz) or DPX500 (500 MHz) spectrometer. The format of the ¹H NMR data below is: chemical shift in ppm down field of the tetramethylsilane reference (multiplicity, coupling constant J in Hz, integration).

### Example 12

### 1-Isopropyl piperazine dihydrochloride

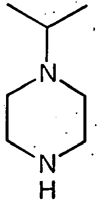

To a solution of *tert*-butyl piperazine-1-carboxylate (100 g) and acetone (48 mL) in CH₂Cl₂ (1 L) was added acetic acid (31 mL) and NaBH(OAc)₃ (170 g). The reaction mixture was stirred for 18 h, then was diluted with 1 N NaOH (500 mL), and extracted with CH₂Cl₂ (500 mL x 2). The combined organic layers were dried (Na₂SO₄) and concentrated to a residue. The residue was dissolved in MeOH (200 mL) and 4 M HCl in 1,4-dioxane (700 mL) was added to the reaction mixture over a period of several hours. After 18 h, the reaction mixture was concentrated to yield a solid, which was washed with Et₂O (500 mL x 2) and dried overnight to yield the title compound as a white solid.

¹H NMR (CD₃OD): 3.76-3.51 (m, 9H), 1.44 (d, J = 6.7 Hz, 6H).

### Example 13

### 4-Formyl-benzoyl chloride

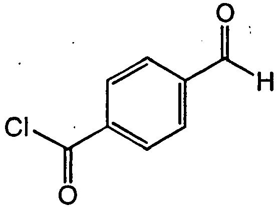

A suspension of (chloromethylene)dimethylammonium chloride (Vilsmeier Reagent; 37.7 g, 0.280 mol) in CH₂Cl₂ (300 mL) at 0°C was treated with 4-carboxybenzaldehyde (40.0 g, 267 mmol) in one portion. The reaction mixture was stirred at 0°C for 30 min, then at room temperature for 2 h. HPLC analysis of an aliquot of the reaction mixture quenched into MeOH indicated consumption of 4-carboxybenzaldehyde. The reaction mixture was filtered through a medium porosity glass frit. The filtrate, containing the title compound, was stored at 0°C, and used in the next step without further manipulation.

### Example 14 (reference example)

### 4-(4-Isopropyl-piperazine-1-carbonyl)-benzaldehyde

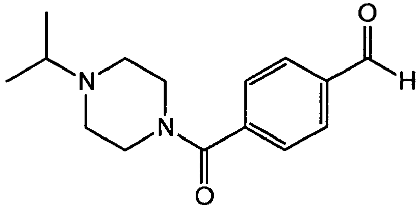

To a suspension of isopropyl piperazine dihydrochloride salt (52.5 g, 262 mmol) (prepared as in Example 12 above) in CH₂Cl₂ was added Et₃N (83.5 g, 827 mmol) and the resulting slurry was stirred at room temperature for 1 h, then at 0°C for 30 min. The reaction mixture was filtered through a medium porosity glass frit and the filtrate was cooled to 0°C. A solution of 4-formyl benzoyl chloride in CH₂Cl₂ was added via an addition funnel in a slow stream over 30 min. The resulting mixture was stirred at 0°C for 30 min, then at room temperature for 2 h. The reaction mixture was cooled to 0°C and filtered through a medium porosity glass frit. The filtrate was washed with H₂O, 0.5 N NaOH, and brine (1 X 400 mL each). The organic layer was dried (Na₂SO₄) and concentrated to yield an oil (59.8 g). Trituration of the oil with anhydrous Et₂O (275 mL), followed by removal of the solvent on a rotary evaporator yielded the title compound as a pale yellow-brown oil.

HPLC: R_{T} = 5.43 min.

### Example 15 (reference example)

### (4-Isopropyl-piperazin-1-yl)-(4-morpholin-4-ylmethyl-phenyl)-methanone

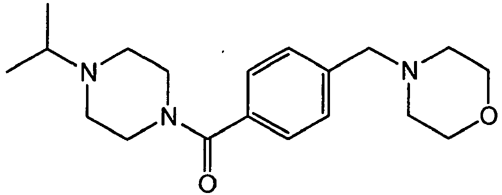

To a solution of 4-(4-isopropyl-piperazine-1-carbonyl)-benzaldehyde (32.0 g, 123 mmol) in THF (650 mL) was added morpholine (21.4 g, 246 mmol), in a slow stream via an addition funnel over 15 min, and the resulting mixture was stirred at room temperature for 40 min. The reaction mixture was treated with NaBH(OAc)₃ (38.4 g, 172 mmol) in portions over 40 min, was stirred at room temperature for 16 h, and then concentrated to a residue. The residue was diluted with EtOAc (400 mL), cooled to 0°C, and treated with 1 N NaOH (250 mL). The biphasic solution stirred at 0°C for 30 min. The phases were separated and the aqueous layer was extracted with EtOAc (2 X 200 mL) and CH₂Cl₂ (2 X 100 mL). The organic layers were combined, washed with brine (1 x 300 mL), dried (Na₂SO₄), and concentrated to yield the title compound as a pale yellow oil.

HPLC: R_{T} = 4.69 min

MS (ESI): calcd. for C₁₉H₂₉N₃O₂, 331.23; m/z found, 332.2 (M+1)

¹H NMR (CDCl₃): 7.36 (s, 4H), 3.79 (br s, 2H), 3.71 (t, J = 4.7 Hz, 4H), 3.51 (s, 2H), 3.44 (br s, 2H), 2.76-2.69 (m, 1H), 2.59 (br s, 2H), 2.44 (t, J = 4.4 Hz, 6H), 1.05 (d, J = 6.5 Hz, 6H).

### Example 16

### (4-Isopropyl-piperazin-1-yl)-(4-morpholin-4-ylmethyl-phenyl)-methanone, bis-maleate salt

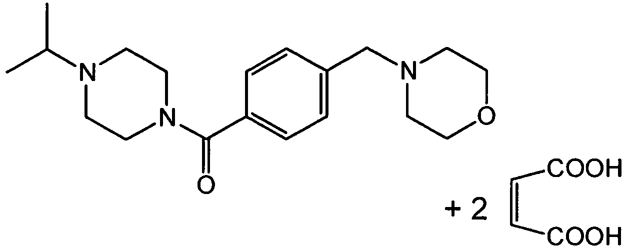

To a solution of (4-isopropyl-piperazin-1-yl)-(4-morpholin-4-ylmethyl-phenyl)-methanone (34.0 g, 102.7 mmol) in absolute EtOH (200 mL) was added, via an addition funnel, a solution of maleic acid (23.9 g, 206 mmol) in absolute EtOH (200 mL) over 15 min. The resulting suspension was stirred at room temperature for 30 min, at 75°C for 1 h, and was then allowed to cool to room temperature over 16 h. The reaction mixture was cooled further to 0°C and was stirred for 2 h. The reaction mixture was diluted with Et₂O (50 mL) and stirred for 30 min. The resulting precipitate was collected by suction filtration, washed with cold EtOH/Et₂O (4:1, 100 mL x 2), and dried in a vacuum oven at 40°C for 20 h to yield the title compound as crude material, as a white solid.

A suspension of the crude (4-isopropyl-piperazin-1-yl)-(4-morpholin-4-ylmethyl-phenyl)-methanone, bis-maleate salt (90.5 g) in absolute EtOH (905 mL) was heated at 75°C for 1 h, and the resulting solution was filtered through a fine porosity glass frit. The filtrate was cooled to room temperature over 20 h, with stirring. The resulting suspension was cooled to 0°C for 2 h, and the precipitate was collected by suction filtration and washed with Et₂O (2 x 200 mL). The solids were dried in a vacuum oven at 40°C for 20 h to yield the title compound as a white crystalline solid.

MP: 148-150 °C

MS (ESI): calcd. for C₁₉H₂₉N₃O₂, 331.23; m/z found, 332.2 (M+1)

¹H NMR (CD₃OD): 7.54-7.48 (m, 4H), 6.26 (s, 4H), 4.23 (s, 2H), 3.85 (br m, 8H), 3.56 (br s, 1H), 3.42-3.32 (br s, 4H), 3.13 (br s, 4H), 1.38 (d, J = 6.6 Hz, 6H). Anal. calcd. for C₂₇H₃₇N₃O₁₀: C, 57.54; H, 6.62; N, 7.46. Found: C, 57.52; H, 6.73; N, 7.54.

### Example 17

### 4-Formyl-benzoyl chloride

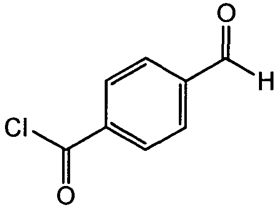

A solution of 4-carboxybenzaldehyde (30.0 g, 0.200 mol) in toluene (300 mL) was treated with thionyl chloride (28.6 g, 0.240 mol) and DMF (1.0 mL). The reaction mixture was heated at 100°C for 2 h, during which time the solids dissolved to yield a pale yellow colored solution. The reaction mixture was cooled to 0°C to yield a solution of the title compound in toluene, which was used without further manipulation.

### Example 18

### (4-Isopropyl-piperazin-1-yl)-(4-piperidin-1-ylmethyl-phenyl)-methanone, bis-maleate salt

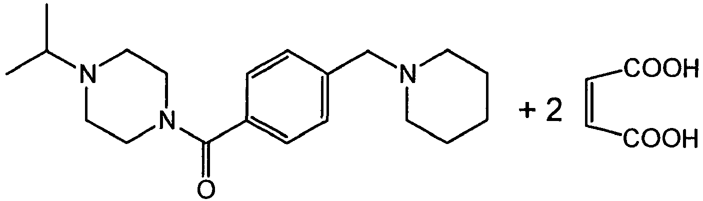

To a mechanically agitated solution of (4-isopropyl-piperazin-1-yl)-(4-piperidin-1-ylmethyl-phenyl)-methanone (40.0 g, 122 mmol) in absolute EtOH (800 mL) was added, via an addition funnel, a solution of maleic acid (28.2 g, 243 mmol) in absolute EtOH (200 mL) over 30 min. The resulting suspension was stirred at room temperature for 16 h, then diluted with Et₂O (200 mL), cooled to 0°C, and stirred for 2 h. The precipitate was collected by suction filtration, washed with cold EtOH/Et₂O (4:1, 3 x 100 mL). The solids were dried under vacuum to yield crude title compound as a white solid.

A mechanically agitated suspension of the crude material (4-isopropyl-piperazin-1-yl)-(4-piperidin-1-ylmethyl-phenyl)-methanone, bis-maleate salt) (89.0 g) in absolute EtOH (1780 mL) was heated at 75°C for 1 h. The resulting pale yellow solution was allowed to cool to room temperature with stirring over 36 h, then diluted with Et₂O (220 mL), cooled to 0°C, and stirred for 3 h. The precipitate was collected by suction filtration, washed with Et₂O (2 x 100 mL).

The solids were dried under vacuum for 16 h to yield the title compound as a white crystalline solid.

| | |
|---|---|
| MP: | 165-167 °C |
| MS (ESI): | calcd. for C₂₀H₃₁N₃O, 329.25; m/z found, 330.2 (M+1) |
| Anal. calcd. for C₂₈H₃₉N₃O₉: | C, 59.88; H, 7.00; N, 7.48. |
| Found: | C, 59.56; H, 7.29; N, 7.40. |

### Example 19 (reference example)

### (4-Cyclopropyl-piperazin-1-yl)-(4-morpholin-4-ylmethyl-phenyl)-methanone

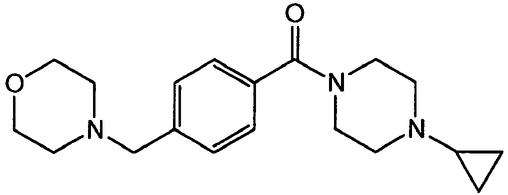

### Step A. 4-(4-Formyl-benzoyl)-piperazine-1-carboxylic acid tert-butyl ester

A suspension of 4-carboxybenzaldehyde (3.10 g) in CH₂Cl₂ was treated sequentially with piperazine-1-carboxylic acid tert-butyl ester (3.6 g), EDCI (3.86 g), HOBt (2.68 g), and 4-dimethylaminopyridine (~0.020 g). After 18 h, the mixture was extracted with 1 N NaOH and then with 1 N HCl. The organic layer was dried (Na₂SO₄) and concentrated to yield the title compound.

MS (ESI): mass calcd. for C₁₇H₂₂N₂O₄, 318.16; m/z found, 219.3 [(M-100)+H]⁺

¹H NMR (CDCl₃): 10.04 (s, 1H), 7.93 (d, J = 8.2, 2H), 7.54 (d, J = 8.1, 2H), 3.82-3.67 (m, 2H), 3.58-3.30 (m, 6H), 1.46 (s, 9H).

### Step B. 4-(4-Morpholin-4-ylmethyl-benzoyl)-piperazine-1-carboxylic acid tert-butyl ester

A solution of 4-(4-formyl-benzoyl)-piperazine-1-carboxylic acid tert-butyl ester (2.06 g) in methanol (100 mL) was treated with morpholine (4 mL) and NaBH(OAc)₃ (6.98 g, in portions over 1 h). After 3 h, the mixture was diluted with saturated aquoues NaHCO₃ and extracted with CH₂Cl₂. The organic layer was dried (Na₂SO₄) and concentrated. The residue was purified by column chromatography (SiO₂) to yield the title compound.

MS (ESI): mass calcd. for C₂₁H₃₁N₃O₄, 389.23; m/z found, 390.4 [M+H]⁺. ¹H NMR (CDCl₃): 7.39-7.33 (m, 4H), 3.75-3.66 (m, 6H), 3.50 (s, 2H), 3.51-3.33 (m, 6H,), 2.45-2.41 (m, 4H), 1.46 (s, 9H).

### Step C. (4-Morpholin-4-ylmethyl-phenyl)-piperazin-1-yl-methanone

A solution of 4-(4-morpholin-4-ylmethyl-benzoyl)-piperazine-1-carboxylic acid tert-butyl ester (1.163 g) in CH₂Cl₂ (10 mL) was treated with trifluoroacetic acid (-4 mL). After 30 min, additional trifluoroacetic acid (5 mL) was added, and the mixture was stirred for a further 2 h. The mixture was diluted with diluted with saturated aquoues NaHCO₃ and extracted with CH₂Cl₂. The organic layer was dried (Na₂SO₄) and concentrated. The residue was purified by column chromatography (SiO₂) to yield the title compound.

MS (ESI): mass calcd. for C₁₆H₂₃N₃O₂, 289.18; m/z found, 290.4 [M+H]^{+ 1}H NMR (CDCl₃): 7.41-7.35 (m, 4H), 3.95-3.70 (m, 6H), 3.52 (s, 2H), 3.09-2.80 (m, 6H), 2.49-2.42 (m, 4H).

### Step D. (4-Cyclopropyl-piperazin-1-yl)-(4-morpholin-4-ylmethyl-phenyl)-methanone

A solution of (4-morpholin-4-ylmethyl-phenyl)-piperazin-1-yl-methanone (0.128 g) in methanol (7.5 mL) was treated with (1-ethoxy-cyclopropoxy)-trimethyl-silane (1.5 mL), acetic acid (0.2 mL), and NaBH₃CN (-400 mg). The mixture was heated at 60°C for 18 h, and then was cooled to room temperature and concentrated. The residue was diluted with 1 N NaOH and extracted with CH₂Cl₂. The organic layer was dried (Na₂SO₄) and concentrated. The residue was purified by column chromatography (SiO₂) to yield the title compound.

MS (ESI): mass calcd. for C₁₉H₂₇N₃O₂, 329.21; m/z found, 330.4 [M+H]⁺

¹H NMR (CDCl₃): 7.36 (s, 4H), 3.79-3.68 (m, 6H), 3.50 (s, 2H), 3.44-3.32 (m, 2H), 2.74-2.61 (m, 2H), 2.60-2.50 (s, 2H), 2.45-2.40 (m, 4H), 1.66-1.62 (m, 1H), 0.49-0.44 (m, 2H), 0.44-0.39 (m, 2H).

### Example 20 (reference example)

### (4-Cyclopropyl-piperazin-1-yl)-(4-morpholin-4-ylmethyl-phenyl)-methanone

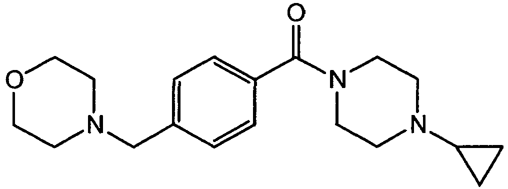

### Step A. tert-Butyl 4-cyclopropylpiperazine-1-carboxylate

A mixture of *tert*-butyl piperazine-1-carboxylate (75.0 g), THF (500 mL), methanol (500 mL), [(1-ethoxycyclopropyl)oxy]trimethylsilane (161 mL), NaBH₃CN (38.0 g), and acetic acid (37 mL) was heated at 60 °C for 5 h. The mixture was cooled to room temperature, treated with water (30 mL) and stirred for 5 min. The mixture was then treated with 1 N NaOH (130 mL) and was further stirred for 15 min. The mixture was concentrated, and the remaining aqueous solution was extracted with CH₂Cl₂ (500 mL). The organic layer was washed with 1 N NaOH (500 mL). The combined aqueous layers were extracted with CH₂Cl₂ (150 mL). The combined organic layers were washed with brine (400 mL), dried (Na₂SO₄), and concentrated to yield the title compound as a white solid.

MS (ESI): mass calcd. for C₁₂H₂₂N₂O₂, 226.17; m/z found, 227.2 [M+H⁺]

¹H NMR (400 MHz, CDCl₃): 3.39 (t, *J* = 5.0 Hz, 4H), 2.55 (t, *J* = 4.9 Hz, 4H), 1.60 (ddd, *J* = 10.3, 6.5, 3.8 Hz, 1H), 1.46 (s, 9H), 0.49-0.38 (m, 4H).

### Step B. 1-Cyclopropylpiperazine dihydrochloride

A solution of *tert*-butyl 4-cyclopropylpiperazine-1-carboxylate (92 g) in 1,4-dioxane (200 mL) was treated with HCl (4 M in 1,4-dioxane, 500 mL) over 10 min while maintaining the temperature below 40°C. After the addition was complete, the mixture was heated at 45°C for 9 h and then was cooled to room temperature. The thick suspension was diluted with hexanes (400 mL) and was cooled to 10°C. The resulting solid was collected by filtration, washed with hexanes, and dried to yield the title compound as a white solid.

MS (ESI): mass calcd. for C₇H₁₄N₂, 126.12; m/z found, 127.0 [M+H] ¹H NMR (400 MHz, D₂O): 3.65 (br t, *J*= 4.7 Hz, 4H), 3.47 (br t, *J* = 5.5 Hz, 4H), 2.85 (br quintet, *J* = 5.8 Hz, 1H), 0.94 (br s, 2H), 0.92 (br s, 2H).

### Step C. 4-(4-Cyclopropyl-piperazine-1-carbonyl)-benzaldehyde

A mixture of 4-formyl-benzoic acid (54.4 g), toluene (500 mL), DMF (3.6 mL), and thionyl chloride (30.4, mL) was heated at 60°C for 2 h and then was cooled to 5°C. In a separate flask; a 5°C mixture of NaOH (50.7 g), water (550 mL), and toluene (150 mL) was treated with 1-cyclopropyl-piperazine dihydrochloride (70.0 g) in portions while the temperature was maintained below 10°C. After the addition was complete, the mixture was cooled to 5°C and treated with the crude acyl chloride solution prepared, as above at a rate such that the temperature did not exceed 10°C. After the addition was complete, the mixture was allowed to warm to room temperature and was stirred overnight. The biphasic mixture was basified to pH ~1.0 with 1 N NaOH (300 mL). The layers were separated and the aqueous layer was extracted with toluene (100 mL x 2). The combined organic layers were washed with brine (200 mL), dried (Na₂SO₄), and concentrated to yield the title compound as pale yellow viscous oil.

HPLC: R_{T} = 5.19 min

MS (ESI): mass calcd. for C₁₅H₁₈N₂O₂, 258.14; m/z found, 258.9 [M+H⁺]

¹H NMR (400 MHz, CDCl₃): 10.1 (s, 1H), 7.94 (pseudo d, *J*= 8.2 Hz, 2H); 7.56 (pseudo d, *J* = 8.1 Hz, 2H), 3.77 (br s, 2H), 3.33 (br s, 2H), 2.71 (br s, 2H), 2.55 (br s, 2H), 1.66 (ddd, *J*= 10.2, 6.6, 3.7 Hz, 1H), 0.52-0.46 (m, 2H), 0.45-0.40 (br s, 2H).

### Step D. (4-Cyclopropyl-piperazin-1-yl)-(4-morpholin-4-ylmethyl-phenyl)-methanone

To a solution of 4-(4-cyclopropyl-piperazine-1-carbonyl)-benzaldehyde (56.0 g) in 1,2-dichloroethane (550 mL) was added morpholine (37.8 mL) dropwise over 5 min. The mixture was cooled to 10°C and was treated with NaBH(OAc)₃ (64.3 g) in portions over 1 h. After a further 2 h, the mixture was warmed to room temperature, and a water bath was used to keep the temperature below 20°C. After 18 h, water (60 mL) was added while the temperature was kept under 20°C by the addition of small amounts of ice. After 20 min, the mixture was basified to pH ~10 with 1 N NaOH (450 mL) and the mixture was stirred for 10 min. The layers were separated, and the organic layer was washed with 1 N NaOH (150 mL). The combined aqueous layers were extracted with CH₂Cl₂ (200 mL). The combined organic layers were washed with brine (200 mL), dried (Na₂SO₄), and concentrated to yield the title compound as pale yellow viscous oil.

HPLC: R_{T} = 4.39 min

MS (ESI): mass calcd. for C₁₉H₂₇N₃O₂, 329.21; m/z found, 330.2 [M+H⁺]

¹H NMR (400 MHz, CDCl₃): 7.35 (br s, 4H), 3.73 (br s, 2H), 3.69 (t, *J* = 4.6 Hz, 4H), 3.50 (s, 2H), 3.37 (br s, 2H), 2.67 (br s, 2H), 2.53 (br s, 2H), 2.43 (t, *J* = 4.2 Hz, 4H), 1.63 (ddd, *J* = 10.3, 6.7, 3.7 Hz, 1H), 0.49-0.43 (m, 2H), 0.42-0.39 (br s, 2H).

¹³C NMR (101 MHz, CDCl₃): 170.6, 140.0, 135.1, 129.5, 127.5, 67.4, 63.4, 54.0, 38.7, 6.3.

### Example 21

### (4-Cyclopropyl-piperazin-1-yl)-(4-morpholin-4-ylmethyl-phenyl)-methanone dihydrochloride salt

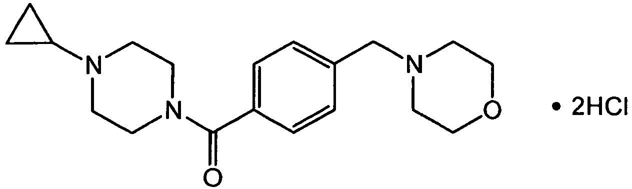

A solution of (4-cyclopropyl-piperazin-1-yl)-(4-morpholin-4-ylmethyl-phenyl)-methanone (68.0 g) in ethanol (400 mL) was heated to 60°C and treated with concentrated HCl (37.8 mL) dropwise over 40 min. A precipitate started to form after ~20 mL of HCl had been added. After the addition was complete, the thick suspension was slowly cooled to 20°C over 3 h. The solid was collected by filtration, washed with ethanol, and dried at 50°C overnight in a vacuum oven to provide the title compound as a white solid.

HPLC: R_{T} = 4.30 min

MS (ESI): mass calcd. for C₁₉H₂₇N₃O₂, 329.21; m/z found, 330.0 [M+H⁺]

¹H NMR (400 MHz, D₂O); 7.64 (pseudo d, *J* = 8.3 Hz, 2H), 7.58 (pseudo d, *J* = 8.3 Hz, 2H), 4.44 (br s, 2H), 4.20-3.10 (m, 16H), 2.88 (ddd, *J* = 11.2, 6.6, 4.8 Hz, 1H), 1.03-0.98 (m, 4H)

¹³C NMR (101 MHz, D₂O): 172.1, 135.3, 132.2, 130.9, 128.0, 64.0, 60.5, 52.6, 52.4, 51.7, 44.8, 39.7, 39.5, 3.9.

### Example 22 (reference example)

### (4-Isopropyl-piperazin-1-yl)-(4-morpholin-4-ylmethyl-phenyl)-methanone

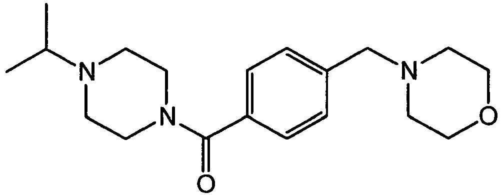

Preparation and analytical data for the title compound was presented in U.S. Patent Application Publication 2004-0110746 A1, published April 21, 2005.

### Example 23

As a specific embodiment of an oral composition, 100 mg of the compound prepared as in Example 21 is formulated with sufficient finely divided lactose to provide a total amount of 580 to 590 mg to fill a size O hard gel capsule.

While the foregoing specification teaches the principles of the present invention, with examples provided for the purpose of illustration, it will be understood that the practice of the invention encompasses all of the usual variations, adaptations and/or modifications as come within the scope of the following claims and their equivalents.

## Claims

1. A process for the preparation of a compound of formula (IIs) 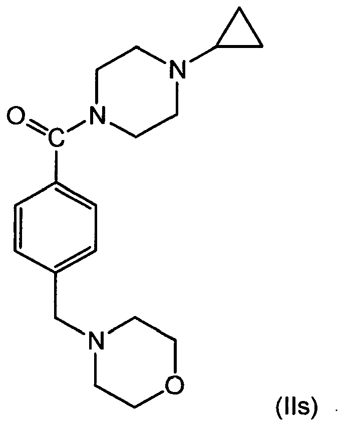 or a hydrate, solvate, or pharmaceutically acceptable salt thereof;
comprising 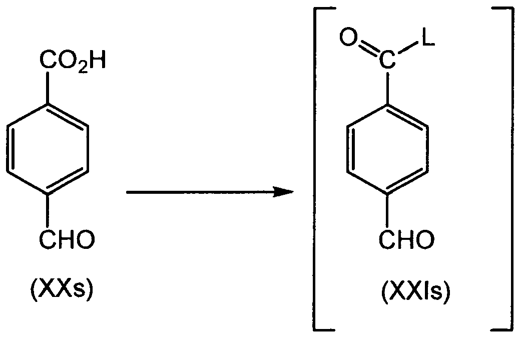 reacting a compound of formula (XXs); in a first organic solvent; to yield the corresponding compound of formula (XXIs), wherein L is a leaving group; and wherein the compound of formula (XXIs) is not isolated; 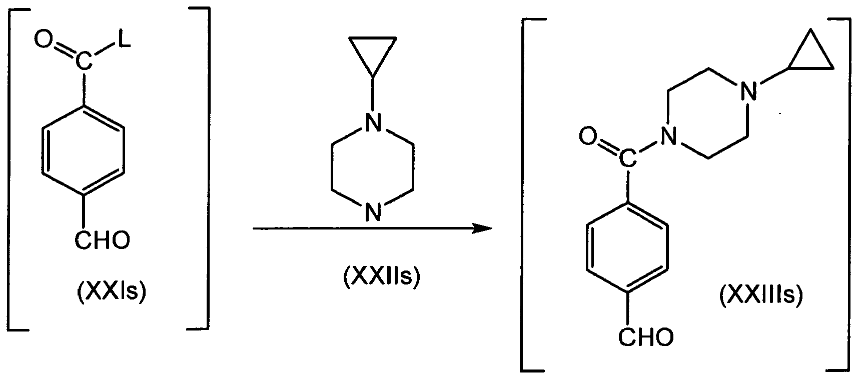 reacting the compound of formula (XXIs) with a compound of formula (XXIIs); in the presence of an organic or inorganic base; in a second organic solvent; to yield the corresponding compound of formula (XXIIIs); wherein the compound of formula (XXIIIs) is not isolated; 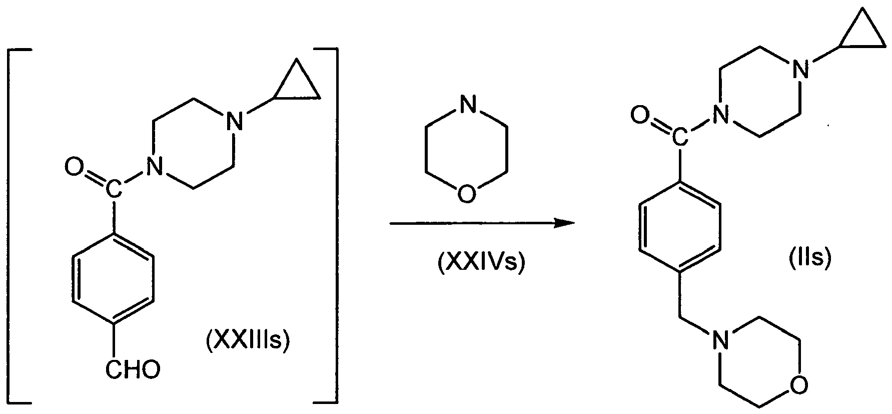 and reacting the compound of formula (XXIIIs) with a compound of formula (XXIVs); in the presence of a reducing agent; in a third organic solvent; to yield the corresponding compound of formula (IIs),
wherein the first organic solvent, the second organic solvent and the third organic solvent are the same,
and wherein L is chloro.

2. A process as in Claim 1, wherein the compound of formula (XXIIs) is present in an amount equal to about one equivalent.

3. A process as in any preceding claim, wherein the compound of formula (XXIVs) is present in an amount greater than about one equivalent; and wherein the reducing agent is present in an amount in the range of from about 1 to about 2 equivalents.

4. A process as in any preceding claim, further comprising reacting the compound of formula (IIs) to yield the corresponding pharmaceutically acceptable salt of the compound of formula (IIs).

## Patentansprüche

1. Verfahren zur Herstellung einer Verbindung der
Formel (IIs) 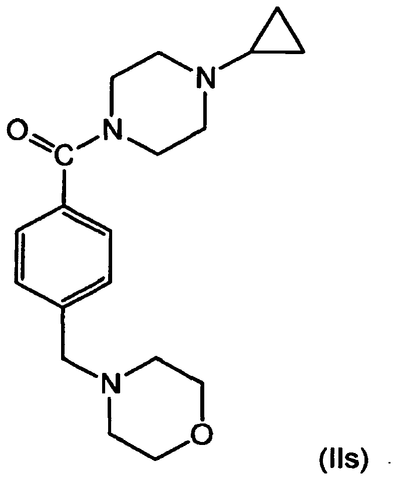 oder eines Hydrats, Solvats oder pharmazeutisch unbedenklichen Salzes davon;
bei dem man 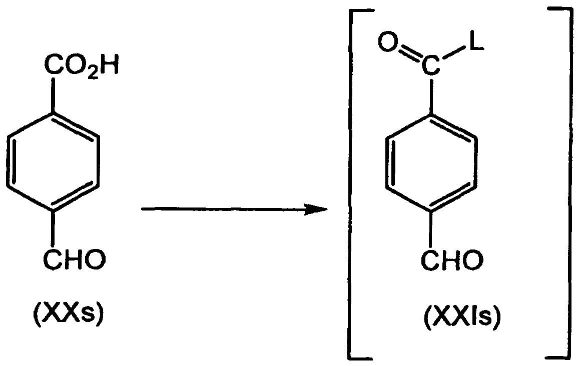 eine Verbindung der Formel (XXs) in einem ersten organischen Lösungsmittel zu der entsprechenden Verbindung der Formel (XXIs), worin L für eine Abgangsgruppe steht, umsetzt, wobei die Verbindung der Formel (XXIs) nicht isoliert wird; 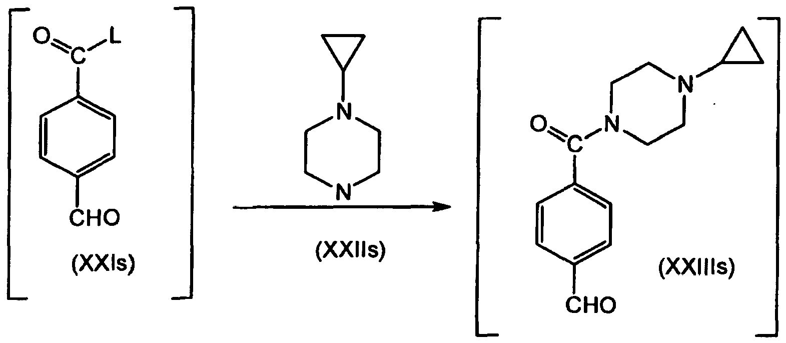 die Verbindung der Formel (XXIs) in Gegenwart einer organischen oder anorganischen Base in einem zweiten organischen Lösungsmittel mit einer Verbindung der Formel (XXIIs) zu der entsprechenden Verbindung der Formel (XXIIIs) umsetzt, wobei die Verbindung der Formel (XXIIIs) nicht isoliert wird; 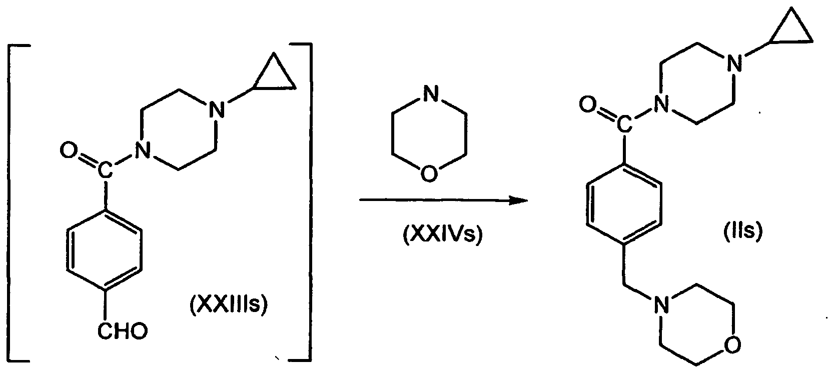 und die Verbindung der Formel (XXIIIs) in Gegenwart eines Reduktionsmittels in einem dritten organischen Lösungsmittel mit einer Verbindung der Formel (XXIVs) zu der entsprechenden Verbindung der Formel (IIs) umsetzt,
wobei das erste organische Lösungsmittel, das zweite organische Lösungsmittel und das dritte organische Lösungsmittel identisch sind,
und wobei L für Chlor steht.

2. Verfahren nach Anspruch 1, bei dem die Verbindung der Formel (XXIIs) in einer Menge von etwa einem Äquivalent vorliegt.

3. Verfahren nach einem der vorhergehenden Ansprüche, bei dem die Verbindung der Formel (XXIVs) in einer Menge von mehr als etwa einem Äquivalent vorliegt und das Reduktionsmittel in einer Menge im Bereich von etwa 1 bis etwa 2 Äuqivalenten vorliegt.

4. Verfahren nach einem der vorhergehenden Ansprüche, bei dem man ferner die Verbindung der Formel (IIs) zu dem entsprechenden pharmazeutisch unbedenklichen Salz der Verbindung der Formel (IIs) umsetzt.

## Revendications

1. Procédé de préparation d'un composé de formule (IIs) 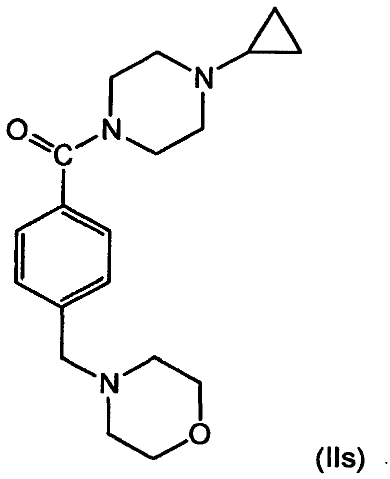 ou d'un hydrate, d'un solvate ou d'un sel pharmaceutiquement acceptable de celui-ci ; comprenant 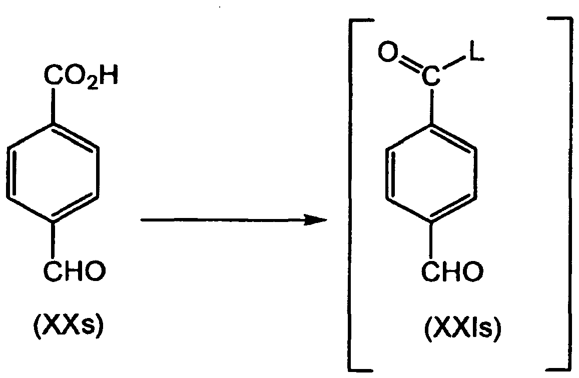 la mise en réaction d'un composé de formule (XXs) ; dans un premier solvant organique ; pour donner le composé correspondant de formule (XXIs), L étant un groupement partant ; et le composé de formule (XXIs) n'étant pas isolé ; 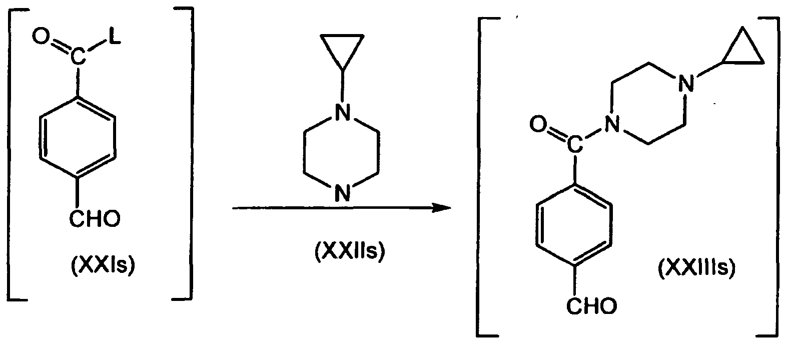 la mise en réaction du composé de formule (XXIs) avec un composé de formule (XXIIs) ; en présence d'une base organique ou minérale ; dans un deuxième solvant organique ; pour donner le composé correspondant de formule (XXIIIs) ; le composé de formule (XXIIIs) n'étant pas isolé ; 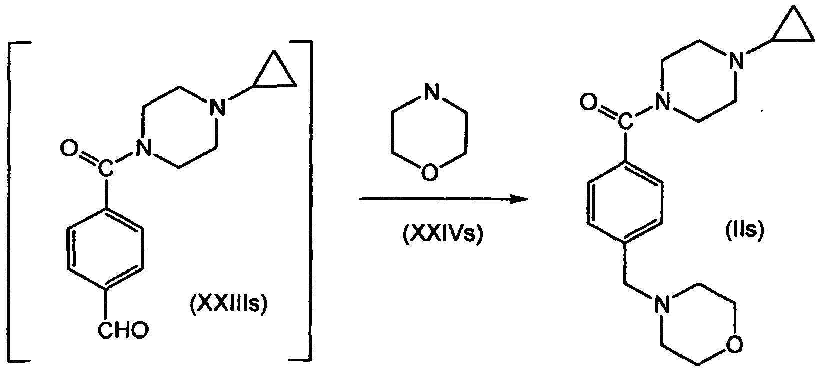 et la mise en réaction du composé de formule (XXIIIs) avec un composé de formule (XXIVs) ; en présence d'un agent de réduction ; dans un troisième solvant organique ; pour donner le composé correspondant (IIs),
le premier solvant organique, le deuxième solvant organique et le troisième solvant organique étant identiques,
et L étant chloro.

2. Procédé selon la revendication 1, **caractérisé en ce que** le composé de formule (XXIIs) est présent dans une quantité égale à environ un équivalent.

3. Procédé selon l'une ou l'autre des revendications précédentes, **caractérisé en ce que** le composé de formule (XXIVs) est présent dans une quantité supérieure à environ un équivalent et l'agent de réduction est présent dans une quantité dans la gamme d'environ 1 à environ 2 équivalents.

4. Procédé selon l'une quelconque des revendications précédentes, comprenant en outre la mise en réaction du composé de formule (IIs) pour donner le sel pharmaceutiquement acceptable correspondant du composé de formule (IIs).
